# EUROPEAN PATENT APPLICATION

(11) **EP 4 141 101 A1**
(43) Date of publication of application: **01.03.2023**
(21) Application number: 21813221.5
(22) Date of filing: 26.01.2021
(51) Int. Cl.: C12M 3/06, C12M 1/36, C12M 1/12, C12M 1/04, C12M 1/00

(54) **CULTURE MEDIUM EXTERNAL CIRCULATION AND RENEWAL APPARATUS FOR BIOREACTOR**

(30) Priority: 26.05.2020 CN 202010457772
(71) Applicant: Alit Biotech (Shanghai) Co., Ltd., Shanghai 201615 (CN)
(72) Inventor: LIU, Yu, Shanghai 201613 (CN); CHEN, Rui, Shanghai 201613 (CN)
(74) Representative: Wang, Bo
(86) International application number: PCT/CN2021/073800
(87) International publication number: WO 2021/238288

(57) **Abstract**

The disclosure discloses an external circulation renewal device of a culture medium for a bioreactor. The bioreactor has a tank containing a mixed solution of animal cells and the culture medium, and the external circulation renewal device of the culture medium comprises: a dialysis component, which is arranged outside the tank and comprises a dialysis filter. The dialysis filter is configured to dialyze harmful metabolites in the culture medium separated from the animal cells into a dialysate. A ventilation component in fluid communication with the dialysis component, which is arranged outside the tank and comprises a gas distributor. The gas distributor is configured to transfer a gas to the culture medium separated from animal cells, and the gas distributor and the dialysis filter are formed as an integral part. The external circulation renewal device of the culture medium integrates the gas exchange function and dialysis function into a whole and makes a compact design.

## Description

### TECHNICAL FIELD

The present disclosure generally relates to the field of bioreactors. More specifically, the present disclosure relates to an external circulation renewal device of culture medium for a bioreactor.

### BACKGROUND

The solubility of oxygen in the culture medium is always one of the most important parameters to design animal cell bioreactors. In general, the oxygen transmission rate is greater than the oxygen consumption rate of animal cells to maintain the normal metabolic level of animal cells.

At present, for the distributors that carry out gas input directly from the inside of the bioreactor tank, most disposable bioreactors adopt the combination of centimeter-scale bubble distributors and micron-scale bubble distributors to immerse the centimeter-scale bubble distributors and micron-scale bubble distributors in a mixed solution of culture medium and animal cells. The diameters of bubbles generated by the centimeter-scale bubble distributors are larger and the mass transfer specific surface area is smaller. Therefore, the centimeter-scale bubble distributors have the better ability to discharge carbon dioxide, but the poor ability to transfer oxygen, and thus they are generally used to disperse excess carbon dioxide bubbles dissolved in the culture medium. The micron-scale bubble distributors are usually made by a sintering method of metal or plastic materials, and its minimum pore diameter may be controlled to 2-20um. Under the same gas flow conditions, the micron-scale bubble distributors produce a large number of bubbles and effectively improve the mass transfer specific surface area. However, too small bubbles produced by the micron-scale bubble distributor may cause significant damage to animal cells (especially stem cells). In addition, in the external circulation device of the culture medium for the bioreactor, the direct contact transfer of gas and solution may produce phenomena such as lots of foam, uneven oxygen transmission, and limited oxygen transmission. Therefore, when designing this kind of device, the mass transfer mode without bubbles has great advantages.

In the design of bioreactor of animal cells, how to eliminate harmful substances in culture medium is also a difficult problem. Animal cells metabolize a large number of by-products with the increased number, and the accumulation of by-products has a great inhibitory effect on the growth and metabolism of animal cells. Therefore, it is necessary to monitor the concentration of such by-products and find ways to discharge such by-products from the culture medium. At present, most disposable bioreactors use perfusion (dilution or gravity sedimentation for liquid exchange) to separate animal cells from the culture medium and inject fresh culture medium. Although this method effectively dilutes the concentration of by-products, it also brings two problems. One is the massive waste of culture medium. Specifically, one to two culture medium volumes are needed to be exchanged daily, and if 14 days is used as a cycle of the whole process, 10-20 culture medium volumes may be lost. The price of animal cell culture medium (especially stem cell culture medium) is generally one thousand to several thousand yuan per liter, and the cost is huge in the large-scale cultivation. The other is the loss of effective growth factors. Specifically, the culture medium is replaced by using a direct dilution method, which not only dilutes the by-products but also dilutes the concentration of growth factors that promote cell growth. Additional growth factors can be added, but the configuration is complex and the cost is very expensive.

### SUMMARY

One of the purposes of the present disclosure is to provide an external circulation renewal device of a culture medium that can overcome at least one defect in the prior art.

The first aspect of the present disclosure relates to an external circulation renewal device of a culture medium for a bioreactor, wherein the bioreactor has a tank containing a mixed solution of animal cells and the culture medium, the external circulation renewal device of the culture medium comprises:
a dialysis component, which is arranged outside the tank and comprises a dialysis filter, wherein the dialysis filter is configured to dialyze harmful metabolites in the culture medium separated from the animal cells into a dialysate, and
a ventilation component in fluid communication with the dialysis component, wherein the dialysis component is arranged outside the tank and comprises a gas distributor, the gas distributor is configured to transfer a gas to the culture medium separated from the animal cells, and the gas distributor and the dialysis filter are formed as an integral part.

In some embodiments, the gas distributor comprises a shell and a diaphragm arranged in the shell, the shell of the gas distributor comprises a hollow inner cavity, and the diaphragm separates the hollow inner cavity of the shell into a culture medium chamber through which the culture medium flows and a gas chamber through which the gas flows.

In some embodiments, the diaphragm is configured to dissolve the gas in the gas chamber into the culture medium in the culture medium chamber without bubbles.

In some embodiments, the diaphragm is arranged to make the culture medium chamber and the gas chamber in a form of an inner cylinder and an outer cylinder nested with each other.

In some embodiments, the diaphragm is in a shape of a cylinder with both ends open and is serrated or wavy along a circumferential direction of the cylinder with both open ends.

In some embodiments, a top end of the diaphragm is fixedly connected to a top wall of the shell, and a bottom end is fixedly connected to a bottom wall of the shell.

In some embodiments, non-woven fabric is configured to adhere to the diaphragm to enhance strength of the diaphragm.

In some embodiments, the gas chamber comprises a ventilation slot arranged along an inner surface of a sidewall of the shell, and the ventilation slot is configured to guide the inlet gas to flow on the entire inner surface of the sidewall.

In some embodiments, the diaphragm is arranged on the inner surface of the sidewall and covers the ventilation slot.

In some embodiments, the sidewall comprises a plurality of convex parts, and the plurality of convex parts protrude radially inward from the inner surface of the sidewall.

In some embodiments, the plurality of convex parts form a support frame for supporting the diaphragm.

In some embodiments, the ventilation slot is formed in a space between the inner surface of the sidewall and side surfaces of adjacent convex parts.

In some embodiments, the diaphragm is fixed to a radial inner surface of the convex parts of the sidewall.

In some embodiments, the ventilation slot is in a serpentine shape winding mainly along a horizontal direction on the entire inner surface of the sidewall.

In some embodiments, the ventilation slot is in a serpentine shape winding mainly along a vertical direction on the entire inner surface of the sidewall.

In some embodiments, the ventilation slot is in a spiral shape extending from the bottom to the top around the entire inner surface of the sidewall.

In some embodiments, the culture medium chamber of the gas distributor is in fluid communication with the tank through a liquid outlet pipe.

In some embodiments, the diaphragm uses a dense membrane or a microporous membrane.

In some embodiments, a pore size of the microporous membrane is configured to make the diaphragm ventilated without bubbles under a certain pressure.

In some embodiments, the pore size of the microporous membrane is less than 0.05 µm.

In some embodiments, the pore size of the microporous membrane is one or more of 0.01 µm, 0.05 um, 0.10 µm, 0.20 µm, and 0.04 µm.

In some embodiments, a thickness of the dense membrane is between 50 µm to 500 µm.

In some embodiments, the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like.

In some embodiments, the diaphragm is processed with hydrophilic and positive charge or negative charge treatment so that it is not easy to be blocked due to being adsorbed by animal cells.

In some embodiments, the ventilation component further comprises a gas inlet unit and a gas outlet unit, wherein the gas inlet unit is in fluid communication with the gas chamber and transmits the inlet gas to the gas chamber, and the outlet unit is in fluid communication with the gas chamber and discharges an undissolved gas in the gas chamber from the gas distributor.

In some embodiments, the gas inlet unit is in fluid communication with the gas chamber through a gas inlet pipe and comprises a gas flow component regulator arranged on the gas inlet pipe, the gas flow component regulator is configured to adjust a component proportion of the inlet gas.

In some embodiments, the gas flow component regulator is one of a mass flowmeter or a gas-proportion regulating valve.

In some embodiments, the inlet gas includes air, oxygen, and carbon dioxide, or includes nitrogen, oxygen, and carbon dioxide.

In some embodiments, the gas outlet unit is in fluid communication with the gas chamber through a gas outlet pipe and comprises a pressure control valve and a sensor arranged in the gas outlet pipe to control gas pressure in the gas chamber.

In some embodiments, the gas pressure in the gas chamber is maintained between 0.01Mpa-0.1Mpa.

In some embodiments, the ventilation component further comprises a dissolved oxygen electrode arranged in the mixed solution, and the dissolved oxygen electrode is configured to detect a dissolved oxygen concentration value in the mixed solution.

In some embodiments, a controller is configured to adjust a proportion of oxygen in the inlet gas of the gas flow component regulator through the dissolved oxygen concentration value detected by the dissolved oxygen electrode, so as to adjust the dissolved oxygen concentration value of the mixed solution in the tank.

In some embodiments, the ventilation component further comprises a pH electrode arranged in the mixed solution, and the pH electrode is configured to detect a pH value in the mixed solution.

In some embodiments, the controller is configured to adjust a proportion of carbon dioxide in the inlet gas of the gas flow component regulator through the pH value detected by the pH electrode, so as to adjust the pH value of the mixed solution.

In some embodiments, the dialysis filter comprises a shell, a filter element arranged in the shell, and a hollow inner cavity, wherein the shell of the dialysis filter is in a shape of a cylinder with a top wall, a bottom wall and a sidewall extending between the top wall and the bottom wall, and the filter element separates the hollow inner cavity of the shell of the dialysis filter into a culture medium chamber through which the culture medium flows and a dialysate chamber through which the dialysate flows.

In some embodiments, the shell of the dialysis filter and the shell of the gas distributor are integrally formed.

In some embodiments, the shell of the dialysis filter and the shell of the gas distributor are formed separately and fixed together by connection.

In some embodiments, an outer cross-section of the shell of the dialysis filter and an outer cross-section of the shell of the gas distributor correspond to each other or do not correspond to each other.

In some embodiments, the culture medium chamber and dialysate chamber of the dialysis filter are in the form of an inner cylinder and an outer cylinder nested with each other.

In some embodiments, the culture medium chamber and dialysate chamber of the dialysis filter are in a form of two half-cylinders adjacent to each other.

In some embodiments, the culture medium chamber of the dialysis filter and the culture medium chamber of the gas distributor are in fluid communication through a connecting pipe.

In some embodiments, the connecting pipe and a corresponding part of the shell of the dialysis filter and/or a corresponding part of the shell of the gas distributor are arranged to be transparent so that a flow state of the culture medium in the connecting pipe can be observed.

In some embodiments, the connecting pipe is substantially in a shape of a funnel, one end of the funnel being connected to the culture medium chamber of the dialysis filter and the other end being connected to the culture medium chamber of the gas distributor.

In some embodiments, the culture medium chamber of the dialysis filter is in fluid communication with the tank through a liquid inlet pipe.

In some embodiments, a pump is arranged on the liquid inlet pipe and is configured to pump the culture medium from the tank to the culture medium chamber of the dialysis filter.

In some embodiments, the dialysis component further comprises a fresh dialysate storage tank and a waste dialysate storage tank, both of which are in fluid communication with the dialysate chamber.

In some embodiments, the pump is arranged on a pipe between the dialysate chamber and the fresh dialysate storage tank or on a pipe between the dialysate chamber and the waste dialysate storage tank, and is configured to drive the dialysate to flow between the fresh dialysate storage tank, the dialysate chamber and the waste dialysate storage tank.

In some embodiments, the filter is arranged on the pipe between the dialysate chamber and the fresh dialysate storage tank for filtering out insoluble particles in the dialysate.

In some embodiments, the external circulation renewal device of the culture medium further comprises a cell separation device configured to separate the culture medium from the animal cells.

In some embodiments, the cell separation device is arranged in the tank and is in fluid communication with the dialysis filter.

The second aspect of the present disclosure relates to an external circulation renewal device of a culture medium for a bioreactor, wherein the bioreactor has a tank containing a mixed solution of animal cells and the culture medium,
the external circulation renewal device of the culture medium comprising a ventilation component, wherein the ventilation component is arranged outside the tank and comprises a gas distributor, the gas distributor is configured to transfer gas to the culture medium separated from the animal cells, the gas distributor includes a shell and a diaphragm arranged in the shell, wherein the shell of the gas distributor includes a hollow inner cavity, the diaphragm separates the hollow inner cavity of the shell into a culture medium chamber through which the culture medium flows and a gas chamber through which the gas flows; and the diaphragm is configured to dissolve the gas in the gas chamber into the culture medium in the culture medium chamber without bubbles.

In some embodiments, the diaphragm is arranged to make the culture medium chamber and the gas chamber in a form of an inner cylinder and an outer cylinder nested with each other.

In some embodiments, the diaphragm is in a shape of a cylinder with both ends open and is serrated or wavy along a circumferential direction of the cylinder with both open ends.

In some embodiments, a top end of the diaphragm is fixedly connected to a top wall of the shell, and a bottom end is fixedly connected to a bottom wall of the shell.

In some embodiments, non-woven fabric is configured to adhere to the diaphragm to enhance strength of the diaphragm.

In some embodiments, the gas chamber comprises a ventilation slot arranged along an inner surface of a sidewall of the shell, and the ventilation slot is configured to guide the inlet gas to flow on the entire inner surface of the sidewall.

In some embodiments, the diaphragm is arranged on the inner surface of the sidewall and covers the ventilation slot.

In some embodiments, the sidewall comprises a plurality of convex parts, and the plurality of convex parts protrude radially inward from the inner surface of the sidewall.

In some embodiments, the plurality of convex parts form a support frame for supporting the diaphragm.

In some embodiments, the ventilation slot is formed in a space between the inner surface of the sidewall and side surfaces of adjacent convex parts.

In some embodiments, the diaphragm is fixed to a radial inner surface of the convex parts of the sidewall.

In some embodiments, the ventilation slot is in a serpentine shape winding mainly along a horizontal direction on the entire inner surface of the sidewall.

In some embodiments, the ventilation slot is in a serpentine shape winding mainly along a vertical direction on the entire inner surface of the sidewall.

In some embodiments, the ventilation slot is in a spiral shape extending from the bottom to the top around the entire inner surface of the sidewall.

In some embodiments, the culture medium chamber of the gas distributor is in fluid communication with the tank through a liquid outlet pipe.

In some embodiments, the diaphragm uses a dense membrane or a microporous membrane.

In some embodiments, a pore size of the microporous membrane is configured to make the diaphragm ventilated without bubbles under a certain pressure.

In some embodiments, the pore size of the microporous membrane is less than 0.05 µm.

In some embodiments, the pore size of the microporous membrane is one or more of 0.01 µm, 0.05 um, 0.10 µm, 0.20 µm, and 0.04 µm.

In some embodiments, a thickness of the dense membrane is between 50 µm to 500 µm.

In some embodiments, the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like.

In some embodiments, the diaphragm is processed with hydrophilic and positive charge or negative charge treatment so that it is not easy to be blocked due to being adsorbed by animal cells.

In some embodiments, the ventilation component further comprises a gas inlet unit and a gas outlet unit, wherein the gas inlet unit is in fluid communication with the gas chamber and transmits the inlet gas to the gas chamber, and the outlet unit is in fluid communication with the gas chamber and discharges an undissolved gas in the gas chamber from the gas distributor.

In some embodiments, the gas inlet unit is in fluid communication with the gas chamber through a gas inlet pipe and comprises a gas flow component regulator arranged on the gas inlet pipe, the gas flow component regulator is configured to adjust the component proportion of the inlet gas.

In some embodiments, the gas flow component regulator is one of a mass flowmeter or a gas-proportion regulating valve.

In some embodiments, the inlet gas includes air, oxygen, and carbon dioxide, or includes nitrogen, oxygen, and carbon dioxide.

In some embodiments, the gas outlet unit is in fluid communication with the gas chamber through a gas outlet pipe and comprises a pressure control valve and a sensor arranged in the gas outlet pipe to control gas pressure in the gas chamber.

In some embodiments, the gas pressure in the gas chamber is maintained between 0.01Mpa-0.1Mpa.

In some embodiments, the ventilation component further comprises a dissolved oxygen electrode arranged in the mixed solution, and the dissolved oxygen electrode is configured to detect a dissolved oxygen concentration value in the mixed solution.

In some embodiments, a controller is configured to adjust a proportion of oxygen in the inlet gas of the gas flow component regulator through the dissolved oxygen concentration value detected by the dissolved oxygen electrode, so as to adjust the dissolved oxygen concentration value of the mixed solution in the tank.

In some embodiments, the ventilation component further comprises a pH electrode arranged in the mixed solution, and the pH electrode is configured to detect a pH value in the mixed solution.

In some embodiments, the controller is configured to adjust a proportion of carbon dioxide in the inlet gas of the gas flow component regulator through the pH value detected by the pH electrode, so as to adjust the pH value of the mixed solution.

In some embodiments, the cell separation device is arranged in the tank and is in fluid communication with the gas distributor.

In some embodiments, the cell separation device is set in the tank and connects to the gas distributor fluid.

Other features and advantages of the subject technology of the present disclosure will be described in the following description and will be apparent in part from the description, or can be learned by practicing the subject technology of the present disclosure. The advantages of the subject technology of the present disclosure will be realized and obtained through the structure especially pointed out in the written disclosure, we claim, and the drawings.

It should be understood that the aforementioned general description and the following detailed description are all exemplary and illustrative, and the purpose of providing further description of the claimed subject technology of the present disclosure.

### BRIEF DESCRIPTION OF THE DRAWINGS

After reading the following specific embodiments in combination with the drawings, various aspects of the present disclosure will be better understood. In the drawings:
FIG. 1 is a schematic diagram illustrating an exemplary external circulation renewal device of a culture medium according to a first embodiment of the present disclosure;
FIG.2A is a perspective diagram illustrating a part of the external circulation renewal device of the culture medium shown in FIG. 1; FIG.2B is an upper cross-sectional diagram illustrating a part of the external circulation renewal device of the culture medium shown in FIG. 1; FIG.2C is a lower cross-sectional diagram illustrating a part of the culture medium external circulation renewal device shown in FIG. 1;
FIGs.3A and 3B are schematic diagrams illustrating exemplary diaphragms of the external circulation renewal device of the culture medium shown in FIG. 1;
FIGs.4A and 4B illustrates an assembled perspective view and a cross-sectional view of a gas distributor in another example of the external circulation renewal device of the culture medium shown in FIG. 1, respectively; FIG.4C illustrates a partial stereoscopic view of a diaphragm of the gas distributor; and
FIG.5 is a schematic diagram illustrating an exemplary external circulation renewal device of the culture medium according to a second embodiment of the present disclosure.

### DETAILED DESCRIPTION

The present disclosure will be described below with reference to the drawings, which show several embodiments of the present disclosure. However, it should be understood that the present disclosure may be presented in a variety of different ways and is not limited to the embodiments described below. In fact, the embodiments described below are intended to make the disclosure of the present disclosure more complete and fully explain the scope of protection of the present disclosure to those skilled in the art. It should also be understood that the embodiments disclosed in the present disclosure can be combined in various ways to provide more additional embodiments.

It should be understood that in all the drawings, the same reference numerals refer to the same elements. In the drawings, the dimensions of some features can be deformed for clarity.

It should be understood that the use of words in the specification are only used to describe specific embodiments and are not intended to limit the present disclosure. Unless otherwise defined, all terms (including technical terms and scientific terms) used in the present disclosure have meanings commonly understood by those skilled in the art. Well-known functions or constructions may not be described in detail for brevity and/or clarity.

As shown in the present disclosure and claim, unless the context clearly prompts the exception, "a", "this", and/or "the" is not specifically singular, and the plural may be included. The terms "comprises," "comprising," "includes," "including" and/or "contain" used in the present disclosure indicate the existence of the claimed features, but do not exclude the existence of one or more other features. The term "and/or" used in the present disclosure includes any and all combinations of one or more of the relevant listed items. The terms "between X and Y" and "between about X and Y" used in the present disclosure shall be interpreted to include X and Y The term "between about X and Y" used in the present disclosure means "between about X and about y", and the term "from about X to Y" used in the present disclosure means "from about X to about Y".

In the present disclosure, when an element is said to be "on", "attached" to another element, "connected" to another element, "coupled" to another element, or "in contact" with another element, the element can be directly on another element, attached to another element, connected to another element, or in contact with another element, or there may be an intermediate element. In contrast, when an element is said to be "directly" on another element, "directly attached" to another element, "directly connected" to another element, "directly coupled" to another element, or "directly in contact" with another element, there may be no intermediate element. In the present disclosure, one feature is arranged to be "adjacent" to another feature, which refers to one feature that has a part overlapping with the adjacent feature or a part above or below the adjacent feature.

In the present disclosure, the spatial relationship terms such as "up", "down", "left", "right", "front", "back", "high", "low" and so on can explain the relationship between one feature and another feature in the drawings. It should be understood that the spatial relationship term includes different orientations of the device in use or operation in addition to the orientations shown in the drawings. For example, when the device in the drawings is reversed, the feature originally described as "below" other features can be described as "above" other features. The device can also be oriented in other ways (rotated 90 degrees or in other directions), and the relative spatial relationship may be explained accordingly.

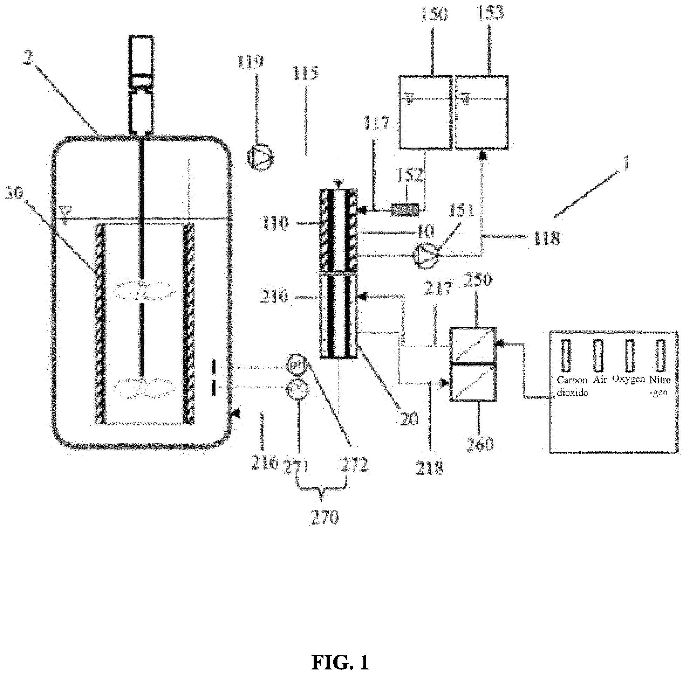

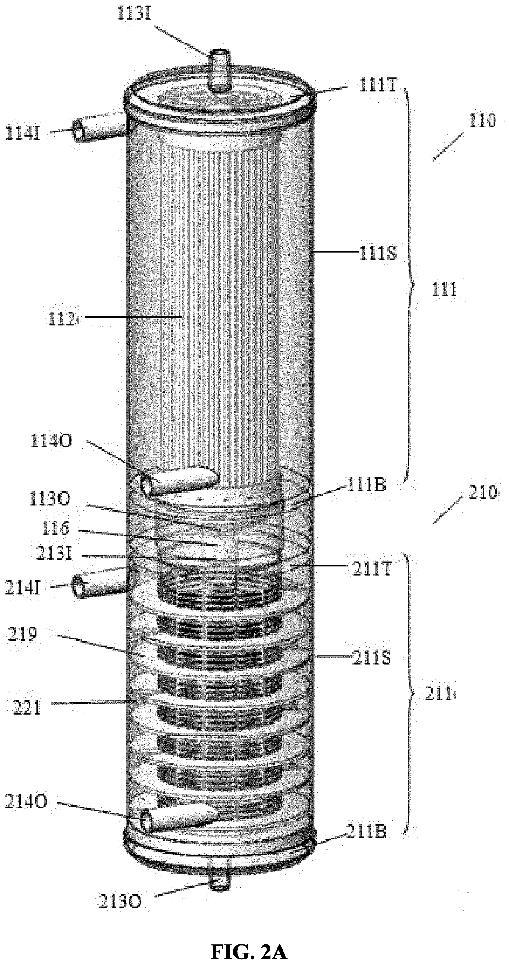

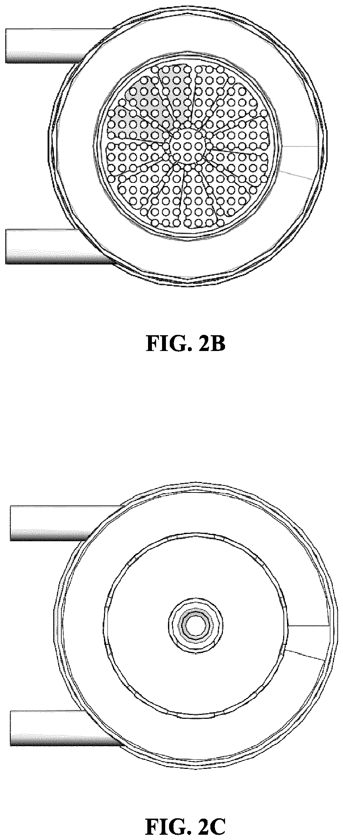

FIG. 1 is a schematic diagram illustrating an exemplary use environment of an external circulation renewal device of a culture medium 1 according to the first embodiment of the present disclosure, and FIGs. 2A, 2B and 2C illustrate a stereoscopic view, an upper cross-sectional view and a lower cross-sectional view of a part of the external circulation renewal device of a culture medium 1, respectively. The external circulation renewal device of the culture medium 1 is used in the field of animal cells culture and microcarrier technology, and is suitable for various small bioreactors (such as disposable animal cell bioreactors). The bioreactor comprises a tank 2. The tank 2 is used to contain a mixed solution of animal cells and a culture medium. The external circulation renewal device of culture medium 1 is used to renew the culture medium separated from animal cells outside the tank 2, such as dialysis of harmful metabolites in the culture medium, transmitting one or more gases to the culture medium, etc.

As shown in the figures, the external circulation renewal device of the culture medium 1 may include a dialysis component 10 and a ventilation component 20 in fluid communication with each other. The dialysis component 10 is used to dialyze harmful metabolites (such as lactic acid, inorganic ammonium, etc.) in the culture medium outside the tank 2. The ventilation component 20 is used to transmit one or more gases (such as a mixture of oxygen, carbon dioxide, and air, or a mixture of oxygen, carbon dioxide, and nitrogen) to the culture medium outside the tank 2.

In some embodiments, the external circulation renewal device of the culture medium 1 also optionally includes any form of a cell separation device 30. The cell separation device 30 is arranged in the tank 2 and is used to separate the culture medium from the animal cells in the tank 2, thereby supplying the culture medium separated from the animal cells to the dialysis component 10, so as to eliminate shear damage of a supply circulating pump to the animal cells.

The dialysis component 10 includes a dialysis filter 110. The dialysis filter 110 is used to dialyze harmful metabolites in the culture medium into the dialysate. The dialysis filter 110 includes a shell 111 and a filter element 112 arranged in the shell 111. The shell 111 is a shape of a cylinder and includes a top wall 111T, a bottom wall 111B, and a sidewall 1115 extending between the top wall 111T and the bottom wall 111B. The top wall 111T, the bottom wall 111B, and the sidewall 111S surround a hollow inner cavity of the shell 111. The cross-section of the shell 111 may be circular, elliptical, triangular, quadrilateral, or any other shape.

The filter element 112 separates the hollow inner cavity of the shell 111 into a culture medium chamber 113 and a dialysate chamber 114. The culture medium chamber 113 is used for the flow of the culture medium, and the dialysate chamber 114 is used for the flow of the dialysate so that the harmful metabolites in the culture medium enter the dialysate of the dialysate chamber 114 from the culture medium chamber 113 through the filter element 112. The filter element 112 may be in a shape of a cylinder with both ends open, the top of the filter element 112 is fixedly connected to the top wall 111T of the shell 111 by means of glue, hot melt, or ultrasonic, the bottom of the filter element 112 is fixedly connected to the bottom wall 111B of the shell 111 by means of glue, hot melt, or ultrasonic. Thus, the filter element 112 separates the hollow inner cavity of the shell 111 into the culture medium chamber 113 and the dialysate chamber 114 in a form of an inner cylinder and an outer cylinder nested with each other, respectively.

The medium chamber 113 is provided with a liquid inlet 113I and a liquid outlet 1130 (for example, on the top wall 111T and the bottom wall 111B of the shell 111). The liquid inlet 113I is in fluid communication with the cell separation device 30 through a liquid inlet pipe 115 to receive the culture medium separated from the animal cells from the cell separation device 30. A pump 119 (e.g., a peristaltic pump) may be provided on the liquid inlet pipe 115 to pump the culture medium separated from the animal cells from the cell separation device 30 into the culture medium chamber 113. The cell separation device 30 is used to separate the culture medium from the animal cells so that the culture medium passing through the liquid inlet pipe 115 does not contain the animal cells, which avoids shear damage to animal cells caused by the pump 119. The liquid outlet 1130 is in fluid communication with the ventilation component 20 through a connecting pipe 116 to deliver the dialyzed medium to the ventilation component 20. The connecting pipe 116 is roughly funnel-shaped, and one end is connected to the liquid outlet 1130 and the other end is connected to a liquid inlet 213I of a culture medium chamber 213 of the ventilation component 20 (which will be described in detail below). In some embodiments, the connecting pipe 116 and its corresponding shell parts on the outside are arranged to be transparent, so that the flow state of the culture medium in the connecting pipe 116 can be observed. The dialysate chamber 114 is provided with a liquid inlet 114I and a liquid outlet 1140 (for example, on the sidewall 111S). The liquid inlet 114I is in fluid communication with a fresh dialysate storage tank 150 through a liquid inlet pipe 117, and the liquid outlet 1140 is in fluid communication with a waste dialysate storage tank 153 through a liquid outlet pipe 118.

In other embodiments, the filter element 112 may also in any other shape. For example, the filter element 112 may be in a sheet shape, and the top of the filter element 112 is fixedly connected to the top wall 111T of the shell 111, while the bottom is fixedly connected to the bottom wall 111B of the shell 111. Thus, the filter element 112 separates the hollow inner cavity of the shell 111 into the culture medium chamber 113 and the dialysate chamber 114 in the form of a half cylinder adjacent to each other.

The fresh dialysate storage tank 150 is used to store fresh dialysate and is in fluid communication with the dialysate chamber 114 of the shell 111 through the liquid inlet pipe 117. The waste dialysate storage tank 153 is used to store waste dialysate and is in fluid communication with the dialysate chamber 114 of the shell 111 through the liquid outlet pipe 118. A pump 151 (for example, a peristaltic pump) may be arranged on the liquid inlet pipe 117 and/or the liquid outlet pipe 118 to drive the dialysate to flow between the fresh dialysate storage tank 150, the dialysate chamber 114, and the waste dialysate storage tank 153. That is, the fresh dialysate is pumped from the fresh dialysate storage tank 150 to the dialysate chamber 114 through the liquid inlet pipe 117, the used waste dialysate is pumped from the dialysate chamber 114 to the waste dialysate storage tank 153 through the liquid outlet pipe 118. A filter 152 is arranged on the liquid inlet pipe 117 to filter out insoluble particles in the dialysate. A controller (not shown) may be electrically connected to the pump 151 and a pump 119 to control the operation of the pumps.

The ventilation component 20 includes a gas distributor 210 and a gas inlet unit 250 and a gas outlet unit 260 in fluid communication with the gas distributor 210. The gas inlet unit 250 is used to deliver a gas to the gas distributor 210. The gas distributor 210 is used to dissolve the gas received from the gas inlet unit 250 into the culture medium. The gas outlet unit 260 is used to exhaust a remaining gas in the gas distributor 210 that is not dissolved in the culture medium from the gas distributor 210. The ventilation component 20 may also include a solution monitoring unit 270 to monitor various parameters of the mixed solution of animal cells and culture medium in the tank 2, such as a dissolved oxygen concentration value and a pH value. The controller (not shown) may be electrically connected to the gas inlet unit 250, the gas outlet unit 260, and the solution monitoring unit 270 to control the gas inlet and gas outlet operations.

The gas distributor 210 and the dialysis filter 110 are formed as an integral part. The gas distributor 210 includes a shell 211 and a diaphragm 212 disposed in the shell 211. The shell 211 accommodates the dialyzed culture medium received from the culture medium chamber 113 of the dialysis component 10 and the gas to be dissolved into the culture medium received from the gas inlet unit 250. The diaphragm 212 transmits and dissolves the gas on one side of the diaphragm 212 into the culture medium on the other side of the diaphragm 212 without bubbles in the shell 211. The shell 211 is in a shape of a cylinder with a top wall 211T, a bottom wall 211B, and a sidewall 211S extending between the top wall 211T and the bottom wall 211B. The top wall 211T, the bottom wall 211B, and the sidewall 211S surround the hollow inner cavity of the shell 211. The cross-section of the shell 211 may be circular, elliptical, triangular, quadrilateral, or any other shape. The cross-section of the shell 211 may be set to correspond to the cross-section of the shell 111 of the dialysis filter 110, or not correspond to the cross-section of the shell 111 of the dialysis filter 110. The sidewall 211S of the shell 211 may extend upward over the top wall 211T of the shell 211, and/or the sidewall 111S of the shell 111 can extend down over the bottom wall 111B of the shell 111, so that the sidewall 211S of the shell 211 and the sidewall 111S of the shell 111 may be formed integrally, or may be formed separately and fixed together by welding (e.g., ultrasonic welding), bonding, snap connection, etc.

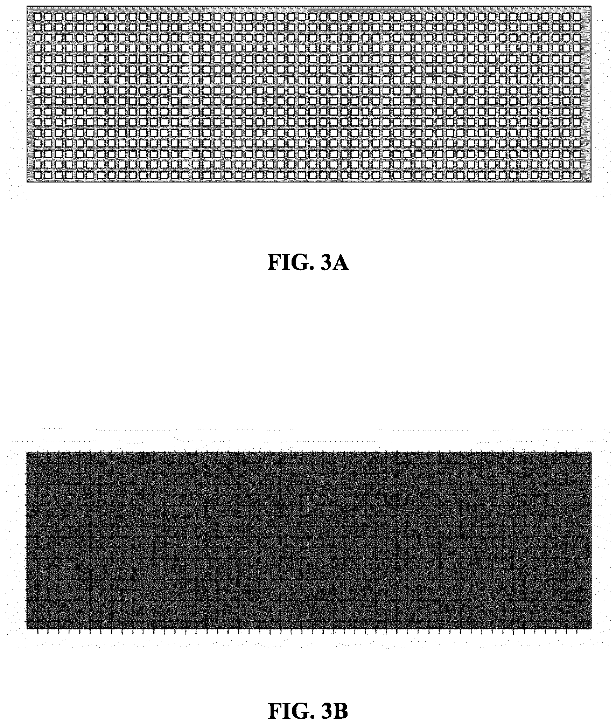

The diaphragm 212 separates the hollow inner cavity of the shell 211 into the culture medium chamber 213 and a gas chamber 214. The culture medium chamber 213 is used for the culture medium to flow through, and the gas chamber 214 is used for the gas to flow through. Therefore, the gas is dissolved into the culture medium of the culture medium chamber 213 in a bubble-free manner by passing through the diaphragm 212 from the gas chamber 214. The diaphragm 212 may be made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like. The diaphragm 212 may be processed with hydrophilic and positive charge or negative charge treatment so that it is not easy to be blocked due to being adsorbed by animal cells. As shown in FIG. 3A, the diaphragm 212 may be in the form of a microporous membrane. The microporous membrane may adopt different pore sizes according to different biological processes (such as stem cells, tumor cells, CHO cells, or microcarrier processes), and the pore sizes are less than 0.05 µm, for example, including but not limited to 0.01µm, 0.05um, 0.10µm, 0.20µm, 0.04µm, etc. Under the above-mentioned pore size conditions, the diaphragm 212 may not produce bubbles when ventilated under a certain pressure. In addition, as shown in FIG. 3B, the diaphragm 212 may be in the form of a dense membrane. The dense membrane may be made of, for example, a silica gel material of PDMS. The dense membrane has no concept of pore sizes, but it is required to be as thin as possible, such as 50 µm to 500 µm.

As shown in FIG.2A, the gas chamber 214 may include a ventilation slot 221 extending on the sidewall 211S of the shell 211. The plurality of convex parts 219 protrude radially inward from the inner surface of the sidewall 21 1S, and the ventilation slot 221 is formed in a space between the inner surface of the sidewall 211S and the side surface of the adjacent convex parts 219. The ventilation slot 221 is used to guide the flow of gas on the entire inner surface of the sidewall 211S to increase the residence time of the gas in the gas distributor 210 and promote gas mass transfer.

The convex parts 219 may form a support frame for supporting the diaphragm 212, thereby keeping the diaphragm 212 flat. The diaphragm 212 may be fixed to the radial inner surfaces of the convex parts 219 of the sidewall 211S by bonding or other means, and covers the ventilation slot 221.

The ventilation slot 221 is in fluid communication with the gas inlet unit 250 through a gas inlet 214I on the sidewall 211S of the shell 211 and with the gas outlet unit 260 through a gas outlet 2140 on the sidewall 211S of the shell 211, thereby forming a gas flow channel through the gas inlet 21I, the ventilation slot 221 and the gas outlet 2140 on the shell 211. The gas may flow into the ventilation slot 221 from the gas inlet 214I, dissolve into the culture medium of the culture medium chamber 213 through the diaphragm 212 above the ventilation slot 221, and the undissolved gas flows out of the ventilation slot 221 through the gas outlet 2140.

The ventilation slot 221 may be in a serpentine shape winding mainly along a horizontal direction on the entire inner surface of the sidewall 211S. The ventilation slot 221 may also be arranged on the sidewall 211S in various other patterns. For example, in one embodiment, the ventilation slot 221 may be in a serpentine shape winding mainly along a vertical direction on the entire inner surface of the sidewall 211S. In another embodiment, the ventilation slot 221 may be in a spiral shape extending from the bottom to the top around the entire inner surface of the sidewall 211S.

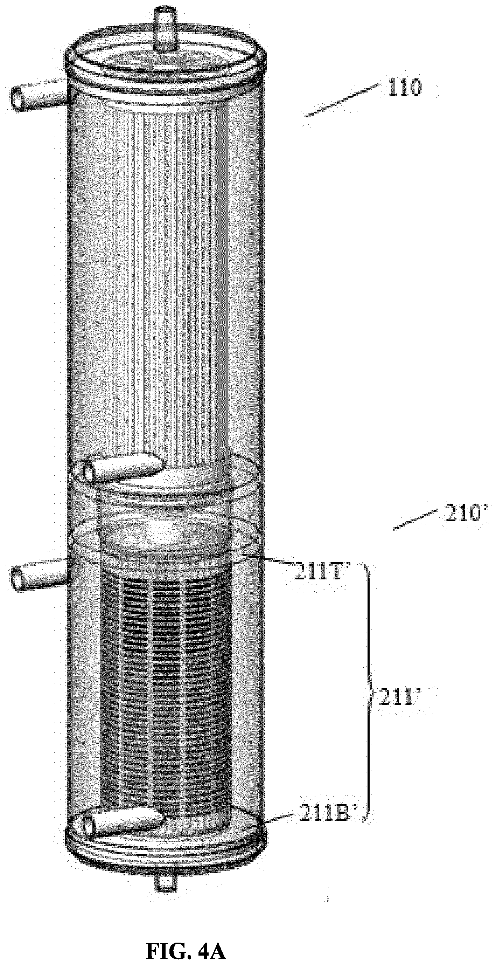

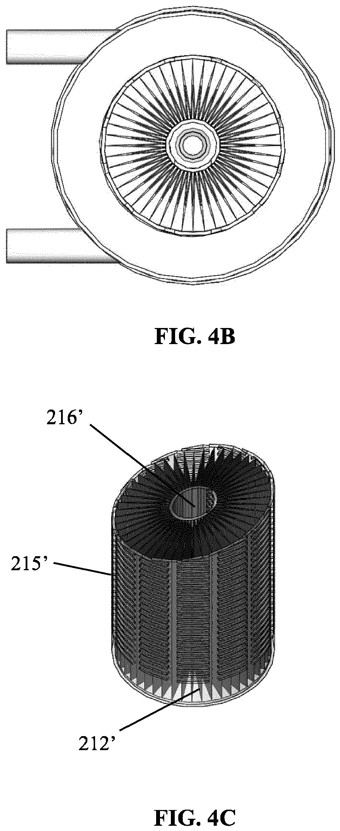

FIG.4A and FIG.4B respectively illustrate an assembled perspective diagram and a cross-sectional diagram of another form of a gas chamber 214' and an associated gas distributor 210', and FIG. 4C shows a perspective diagram of a diaphragm 212' of the gas distributor 210' (the upper half is removed to see the internal structure). As shown in the figures, the diaphragm 212' may be formed into a cylinder with both ends open, and a sidewall of the cylinder may be serrated or wavy. The top of the diaphragm 212' is fixedly connected to a top wall 211T' of the shell 211' by means of glue, hot melt, or ultrasonic, while the bottom is fixedly connected to a bottom wall 211B' of the shell 211' by means of glue, hot melt, or ultrasonic. Thus, the diaphragm 212' separates the hollow inner cavity of the shell 211 into a medium chamber 213' and a gas chamber 214' in the form of an inner cylinder and an outer cylinder nested with each other. Non-woven fabric may be bonded to the entire surface of the diaphragm 212' by bonding, welding, etc., so as to enhance the strength of the diaphragm 212' in the circumferential and vertical directions of the cylinder of the diaphragm, thereby preventing the collapse of the diaphragm 212'. In some embodiments, cylindrical supports 215' and 216' may be respectively added to a radial inner side and an outer side of the diaphragm 212' to support the diaphragm 212'.

Return to FIG.1 and FIG.2A, the culture medium chamber 213 is provided with the liquid inlet 213I and the liquid outlet 2130, respectively (for example, on the top wall 211T and the bottom wall 211B of the shell 211). The liquid inlet 213I is in fluid communication with the culture medium chamber 113 of the dialysis component 10 through the connecting pipe 116 to receive the dialyzed culture medium from the dialysis component 10. The liquid outlet 2130 is in fluid communication with the tank 2 through a liquid outlet pipe 216 to transport the culture medium after dialysis treatment and gas transmission back to the tank 2. The gas chamber 214 is provided with the gas inlet 214I and the gas outlet 2140 (for example, on the top wall 211T, the bottom wall 211B, or the sidewall 211S). The gas inlet 214I is in fluid communication with the gas inlet unit 250 through the gas inlet pipe 217, and the gas outlet 2140 is in fluid communication with the gas outlet unit 260 through the gas outlet pipe 218 to allow the gas to flow through the gas chamber 214.

The gas inlet unit 250 is connected to the gas inlet 214I through the gas inlet pipe 217 and is arranged outside the gas distributor 210. The gas inlet unit 250 transmits the gas from the outside to the ventilation slot 221 in a controlled manner. The gas inlet unit 250 includes a gas flow component regulator (such as a mass flowmeter or a gas-proportion regulating valve) arranged on the gas inlet pipe 217. The gas flow component regulator regulates the flow and component proportion of the inlet gas. In this embodiment, the inlet gas may be mainly composed of air, oxygen, and carbon dioxide, or mainly composed of nitrogen, oxygen, and carbon dioxide. The gas flow component regulator is controlled by the controller to adjust the proportion of oxygen and carbon dioxide in the total flow in real-time, so as to adjust the dissolved oxygen concentration and the pH value in the mixed solution.

The gas outlet unit 260 is connected to the gas outlet 2140 through the gas outlet pipe 218 and is arranged outside the gas distributor 210. The gas outlet unit 260 discharges the undissolved gas in the ventilation slot 221 in a controlled manner to prevent the pressure increase in the gas distributor 210 from affecting the gas control of the gas inlet unit 250. The gas outlet unit 260 includes a pressure control valve and a sensor arranged in the gas outlet pipe 218. The pressure control valve and sensor are controlled by the controller to adjust the gas pressure (generally maintained between 0.01Mpa-0.1Mpa) in the ventilation slot 221 of the gas distributor 210, so as to maintain the mass transfer efficiency of the diaphragm 212.

The solution monitoring unit 270 includes a dissolved oxygen electrode 271 and a pH electrode 272 electrically connected to the controller. The dissolved oxygen electrode 271 and the pH electrode 272 may be arranged in a mixed solution of the cell and the culture medium (or other liquids) in the tank 2. The dissolved oxygen electrode 271 is used to detect the dissolved oxygen concentration value in the mixed solution and feed it back to the controller. The pH electrode 272 is used for determining the pH value of the mixed solution and fed back to the controller. The controller adjusts the oxygen proportion of the inlet gas in the gas flow component regulator of the gas inlet unit 250 in real-time through the dissolved oxygen concentration value detected by the dissolved oxygen electrode 271 of the solution monitoring unit 270. For example, if the dissolved oxygen of the mixed solution is set to no less than 40% during the culture process, with the increase of the number of animal cells in the mixed solution, the oxygen flow also needs to be increased, and the oxygen proportion in the total gas flow also increases in order to maintain the same dissolved oxygen. In addition, the controller adjusts the proportion of carbon dioxide of the inlet gas in the gas flow component regulator of the gas inlet unit 250 through the pH value detected by the pH electrode 272 of the solution monitoring unit 270, so as to adjust the pH value of the mixed solution. For example, if the pH value of the mixed solution is set to a constant value during the culture process, with the continuous increase of pH during the culture process, the flow of carbon dioxide also needs to be increased, and the proportion of carbon dioxide in the total gas flow also increases in order to maintain a constant pH value.

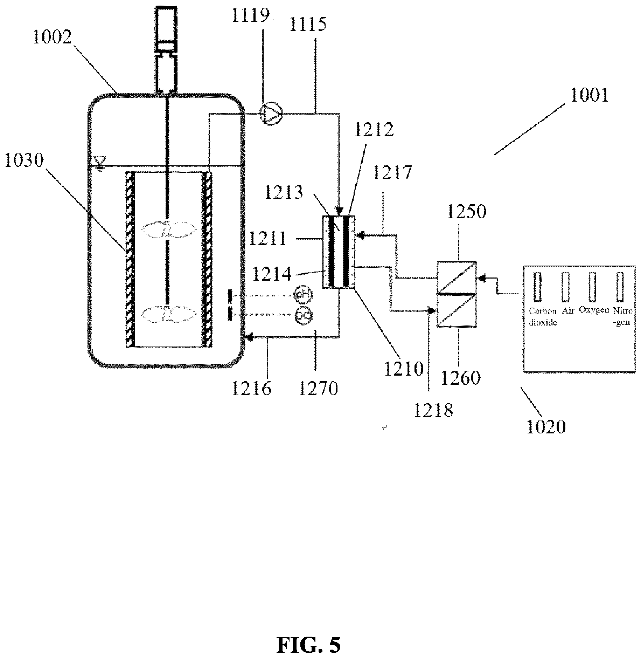

An external circulation renewal device of a culture medium 1001 according to the second embodiment of the present disclosure will be described below with reference to FIG.5. The external circulation renewal device of the culture medium 1001 can represent the same or similar structure with the reference mark in the external circulation renewal device of the culture medium 1 plus 1000.

The external circulation renewal device of the culture medium 1001 is used in the field of animal cell culture and microcarrier technology, and is suitable for various small bioreactors (such as a disposable animal cell bioreactor). The bioreactor includes a tank 1002. The tank 1002 is used to accommodate a mixed solution of animal cells and the culture medium. The external circulation renewal device of the culture medium 1001 is used to renew a culture medium separated from animal cells outside the tank 1002, such as transmitting one or more gases to the culture medium. The main difference between the external circulation renewal device of the culture medium 1001 and the external circulation renewal device of the culture medium 1 is that the external circulation renewal device of the culture medium 1001 may not include a dialysis component.

As shown in FIG.5, the external circulation renewal device of the culture medium 1001 may include a ventilation component 1020. The ventilation component 1020 is used to transmit one or more gases (such as a mixture of oxygen, carbon dioxide, and air, or a mixture of oxygen, carbon dioxide, and nitrogen) to the culture medium outside the tank 1002. In some embodiments, the external circulation renewal device of the culture medium 1001 also optionally includes a cell separation device 1030 in any form. The cell separation device 1030 is arranged in the tank 1002 and is used to separate the culture medium from the animal cells in the tank 1002 to supply the culture medium separated from the animal cells to the ventilation component 1020, so as to eliminate the shear damage of the supply circulating pump to the animal cells.

The ventilation component 1020 includes a gas distributor 1210, a gas inlet unit 1250 in fluid communication with the gas distributor 1210, and a gas outlet unit 1260 in fluid communication with the gas distributor 1210. The gas inlet unit 1250 is used to transmit gas to the gas distributor 1210. The gas distributor 1210 is used to dissolve the gas received from the gas inlet unit 1250 into the culture medium. The gas outlet unit 1260 is used to discharge the remaining gas in the gas distributor 210 that is not dissolved in the culture medium from the gas distributor 1210. The ventilation component 1020 may also include a solution monitoring unit 1270 to monitor various parameters of the mixed solution of animal cells and culture medium in the tank 1002, such as the dissolved oxygen concentration value and the pH value. A controller (not shown) may be electrically connected to the gas inlet unit 1250, the gas outlet unit 1260, and the solution monitoring unit 1270 to control the gas inlet and outlet operations.

The gas distributor 1210 includes a shell 1211 and a diaphragm 1212 disposed in the shell 1211. The shell 1211 accommodates the culture medium separated from animal cells received from the cell separation device 1030 and the gas to be dissolved into the culture medium received from the gas inlet unit 1250. The diaphragm 1212 transmits and dissolves the gas on one side of the shell 1211 into the culture medium on the other side without bubbles.

The diaphragm 1212 separates the hollow inner cavity of the shell 1211 into a culture medium chamber 1213 and a gas chamber 1214. The culture medium chamber 1213 is used for the culture medium to flow through, and the gas chamber 1214 is used for the gas to flow through. Therefore, the gas passes through the diaphragm 1212 from the gas chamber 1214 and is dissolved into the culture medium of the culture medium chamber 1213 in a bubble-free manner.

A liquid inlet of the culture medium chamber 1213 is in fluid communication with the cell separation device 1030 through a connecting pipe 1115 to receive the culture medium separated from animal cells from the cell separation device 1030. A pump 1119 (e.g., a peristaltic pump) may be arranged on the liquid inlet pipe 1115 to pump the culture medium separated from animal cells from the cell separation device 1030 to the culture medium chamber 1213. The cell separation device 1030 is used to separate the culture medium from animal cells, so that the culture medium passing through the liquid inlet pipe 1115 does not contain animal cells, which avoids shear damage to animal cells caused by the pump 1119. A liquid outlet of the medium chamber 1213 is in fluid communication with the tank 1002 through a liquid outlet pipe 1216 to transport the gas-transported medium back to the tank 1002. The gas inlet of the gas chamber 1214 is in fluid communication with the gas inlet unit 1250 through a gas inlet pipe 1217, and the gas outlet is in fluid communication with the gas outlet unit 1260 through a gas outlet pipe 1218 to make the gas flow through the gas chamber 1214.

The structure and function of the gas distributor 1210 are similar to the structure and function of the gas distributor 210 or similar to the structure and function of the gas distributor 210'. The structures and functions of the gas inlet unit 1250, the gas outlet unit 1260 and the solution monitoring unit 1270 are similar to the structures and functions of the gas inlet unit 250, the gas outlet unit 260, and the solution monitoring unit 270. Therefore, the structures and functions of the gas distributor 1210, the gas inlet unit 1250, the gas outlet unit 1260 and the solution monitoring unit 1270 may be understood with reference to the first embodiment.

According to the external circulation renewal device of the culture medium of the present disclosure, based on the cell separation device in the bioreactor tank, the culture medium separated from the animal cells is circulated and renewed, and the shear damage to the animal cells by the circulating pump is reduced.

The external circulation renewal device of the culture medium according to the present disclosure integrates the gas exchange function and the dialysis function into a whole, has a compact design, reduces the volume of the device, and reduces the production cost.

The external circulation renewal device of the culture medium according to the present disclosure uses a microporous membrane or a dense membrane to dissolve the gas, which is more conducive to the transmission effect of the gas. The external circulation renewal device of the culture medium according to the present disclosure does not generate phenomena such as lots of foam, uneven oxygen transmission, and limited oxygen transmission.

Although the exemplary embodiments of the present disclosure have been described, those skilled in the art should understand that various changes and amendments can be made to the exemplary embodiments of the present disclosure without departing from the spirit and scope of the present disclosure in essence. Therefore, all changes and amendments contained in the present disclosure are within the scope of protection. The present disclosure is defined by additional claims, and equivalents of these claims are also included.

## Claims

1. An external circulation renewal device of a culture medium for a bioreactor, wherein the bioreactor has a tank containing a mixed solution of animal cells and the culture medium, the external circulation renewal device of the culture medium comprises:
a dialysis component, which is arranged outside the tank and comprises a dialysis filter, wherein the dialysis filter is configured to dialyze harmful metabolites in the culture medium separated from the animal cells into a dialysate, and
a ventilation component in fluid communication with the dialysis component, wherein the ventilation component is arranged outside the tank and comprises a gas distributor, the gas distributor is configured to transfer a gas to the culture medium separated from the animal cells, and the gas distributor and the dialysis filter are formed as an integral part.

2. The external circulation renewal device of the culture medium according to claim 1, wherein the gas distributor comprises a shell and a diaphragm arranged in the shell, the shell of the gas distributor comprises a hollow inner cavity, and the diaphragm separates the hollow inner cavity of the shell into a culture medium chamber through which the culture medium flows and a gas chamber through which the gas flows.

3. The external circulation renewal device of the culture medium according to claim 2, wherein the diaphragm is configured to dissolve the gas in the gas chamber into the culture medium in the culture medium chamber without bubbles.

4. The external circulation renewal device of the culture medium according to claim 2, wherein the diaphragm is arranged to make the culture medium chamber and the gas chamber in a form of an inner cylinder and an outer cylinder nested with each other.

5. The external circulation renewal device of the culture medium according to claim 4, wherein the diaphragm is in a shape of a cylinder with both ends open and is serrated or wavy along a circumferential direction of the cylinder with both open ends.

6. The external circulation renewal device of the culture medium according to claim 4, wherein a top end of the diaphragm is fixedly connected to a top wall of the shell, and a bottom end is fixedly connected to a bottom wall of the shell.

7. The external circulation renewal device of the culture medium according to claim 4, wherein non-woven fabric is configured to adhere to the diaphragm to enhance strength of the diaphragm.

8. The external circulation renewal device of the culture medium according to claim 2, wherein the gas chamber comprises a ventilation slot arranged along an inner surface of a sidewall of the shell, and the ventilation slot is configured to guide an inlet gas to flow on the entire inner surface of the sidewall.

9. The external circulation renewal device of the culture medium according to claim 8, wherein the diaphragm is arranged on the inner surface of the sidewall and covers the ventilation slot.

10. The external circulation renewal device of the culture medium according to claim 8, wherein the sidewall comprises a plurality of convex parts, and the plurality of convex parts protrude radially inward from the inner surface of the sidewall.

11. The external circulation renewal device of the culture medium according to claim 10, wherein the plurality of convex parts form a support frame for supporting the diaphragm.

12. The external circulation renewal device of the culture medium according to claim 10, wherein the ventilation slot is formed in a space between the inner surface of the sidewall and side surfaces of adjacent convex parts.

13. The external circulation renewal device of the culture medium according to claim 10, wherein the diaphragm is fixed to a radial inner surface of the convex parts of the sidewall.

14. The external circulation renewal device of the culture medium according to claim 8, wherein the ventilation slot is in a serpentine shape winding mainly along a horizontal direction on the entire inner surface of the sidewall.

15. The external circulation renewal device of the culture medium according to claim 8, wherein the ventilation slot is in a serpentine shape winding mainly along a vertical direction on the entire inner surface of the sidewall.

16. The external circulation renewal device of the culture medium according to claim 8, wherein the ventilation slot is in a spiral shape extending from the bottom to the top around the entire inner surface of the sidewall.

17. The external circulation renewal device of the culture medium according to claim 2, wherein the culture medium chamber of the gas distributor is in fluid communication with the tank through a liquid outlet pipe.

18. The external circulation renewal device of the culture medium according to any one of claims 1-17, wherein the diaphragm uses a dense membrane or a microporous membrane.

19. The external circulation renewal device of the culture medium according to claim 18, wherein a pore size of the microporous membrane is configured to make the diaphragm ventilated without bubbles under a certain pressure.

20. The external circulation renewal device of the culture medium according to claim 19, wherein the pore size of the microporous membrane is less than 0.05 µm.

21. The external circulation renewal device of the culture medium according to claim 20, wherein the pore size of the microporous membrane is one or more of 0.01 µm, 0.05 um, 0.10 µm, 0.20 µm, and 0.04 µm.

22. The external circulation renewal device of the culture medium according to claim 18, wherein a thickness of the dense membrane is between 50 µm to 500 µm.

23. The external circulation renewal device of the culture medium according to any one of claims 1-17, wherein the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like.

24. The external circulation renewal device of the culture medium according to any one of claims 1-17, wherein the diaphragm is processed with hydrophilic and positive charge or negative charge treatment so that it is not easy to be blocked due to being adsorbed by animal cells.

25. The external circulation renewal device of the culture medium according to any one of claims 2-17, wherein the ventilation component further comprises a gas inlet unit and a gas outlet unit, wherein the gas inlet unit is in fluid communication with the gas chamber and transmits the inlet gas to the gas chamber, and the outlet unit is in fluid communication with the gas chamber and discharges an undissolved gas in the gas chamber from the gas distributor.

26. The external circulation renewal device of the culture medium according to claim 25, wherein the gas inlet unit is in fluid communication with the gas chamber through a gas inlet pipe and comprises a gas flow component regulator arranged on the gas inlet pipe, the gas flow component regulator being configured to adjust a component proportion of the inlet gas.

27. The external circulation renewal device of the culture medium according to claim 26, wherein the gas flow component regulator is one of a mass flowmeter or a gas-proportion regulating valve.

28. The external circulation renewal device of the culture medium according to claim 25, wherein the inlet gas includes air, oxygen, and carbon dioxide, or includes nitrogen, oxygen, and carbon dioxide.

29. The external circulation renewal device of the culture medium according to claim 25, wherein the gas outlet unit is in fluid communication with the gas chamber through a gas outlet pipe and comprises a pressure control valve and a sensor arranged in the gas outlet pipe to control gas pressure in the gas chamber.

30. The external circulation renewal device of the culture medium according to claim 29, wherein the gas pressure in the gas chamber is maintained between 0.01Mpa-0. 1Mpa.

31. The external circulation renewal device of the culture medium according to claim 26, wherein the ventilation component further comprises a dissolved oxygen electrode arranged in the mixed solution, and the dissolved oxygen electrode is configured to detect a dissolved oxygen concentration value in the mixed solution.

32. The external circulation renewal device of the culture medium according to claim 31, wherein a controller is configured to adjust a proportion of oxygen in the inlet gas of the gas flow component regulator through the dissolved oxygen concentration value detected by the dissolved oxygen electrode, so as to adjust the dissolved oxygen concentration value of the mixed solution in the tank.

33. The external circulation renewal device of the culture medium according to claim 26, wherein the ventilation component further comprises a pH electrode arranged in the mixed solution, and the pH electrode is configured to detect a pH value in the mixed solution.

34. The external circulation renewal device of the culture medium according to claim 33, wherein the controller is configured to adjust a proportion of carbon dioxide in the inlet gas of the gas flow component regulator through the pH value detected by the pH electrode, so as to adjust the pH value of the mixed solution.

35. The external circulation renewal device of the culture medium according to any one of claims 2-17, wherein the dialysis filter comprises a shell, a filter element arranged in the shell, and a hollow inner cavity, the shell of the dialysis filter is in a shape of a cylinder with a top wall, a bottom wall and a sidewall extending between the top wall and the bottom wall, and the filter element separates the hollow inner cavity of the shell of the dialysis filter into a culture medium chamber through which the culture medium flows and a dialysate chamber through which the dialysate flows.

36. The external circulation renewal device of the culture medium according to claim 35, wherein the shell of the dialysis filter and the shell of the gas distributor are integrally formed.

37. The external circulation renewal device of the culture medium according to claim 35, wherein the shell of the dialysis filter and the shell of the gas distributor are formed separately and fixed together by connection.

38. The external circulation renewal device of the culture medium according to claim 35, wherein an outer cross-section of the shell of the dialysis filter and an outer cross-section of the shell of the gas distributor correspond to each other or do not correspond to each other.

39. The external circulation renewal device of the culture medium according to claim 35, wherein the culture medium chamber and dialysate chamber of the dialysis filter are in the form of an inner cylinder and an outer cylinder nested with each other.

40. The external circulation renewal device of the culture medium according to claim 35, wherein the culture medium chamber and dialysate chamber of the dialysis filter are in a form of two half-cylinders adjacent to each other.

41. The external circulation renewal device of the culture medium according to claim 35, wherein the culture medium chamber of the dialysis filter and the culture medium chamber of the gas distributor are in fluid communication through a connecting pipe.

42. The external circulation renewal device of the culture medium according to claim 41, wherein the connecting pipe and a corresponding part of the shell of the dialysis filter and/or a corresponding part of the shell of the gas distributor are arranged to be transparent so that a flow state of the culture medium in the connecting pipe can be observed.

43. The external circulation renewal device of the culture medium according to claim 41, wherein the connecting pipe is substantially in a shape of a funnel, one end of the funnel being connected to the culture medium chamber of the dialysis filter and the other end being connected to the culture medium chamber of the gas distributor.

44. The external circulation renewal device of the culture medium according to claim 35, wherein the culture medium chamber of the dialysis filter is in fluid communication with the tank through a liquid inlet pipe.

45. The external circulation renewal device of the culture medium according to claim 44, wherein a pump is arranged on the liquid inlet pipe and is configured to pump the culture medium from the tank to the culture medium chamber of the dialysis filter.

46. The external circulation renewal device of the culture medium according to claim 35, wherein the dialysis component further comprises a fresh dialysate storage tank and a waste dialysate storage tank, both of which are in fluid communication with the dialysate chamber.

47. The external circulation renewal device of the culture medium according to claim 46, wherein the pump is arranged on a pipe between the dialysate chamber and the fresh dialysate storage tank or on a pipe between the dialysate chamber and the waste dialysate storage tank, and is configured to drive the dialysate to flow between the fresh dialysate storage tank, the dialysate chamber and the waste dialysate storage tank.

48. The external circulation renewal device of the culture medium according to claim 46, wherein the filter is arranged on the pipe between the dialysate chamber and the fresh dialysate storage tank for filtering out insoluble particles in the dialysate.

49. The external circulation renewal device of the culture medium according to any one of claims 1-17, wherein the external circulation renewal device of the culture medium further comprises a cell separation device configured to separate the culture medium from the animal cells.

50. The external circulation renewal device of the culture medium according to claim 49, wherein the cell separation device is arranged in the tank and is in fluid communication with the dialysis filter.

51. An external circulation renewal device of a culture medium for a bioreactor, wherein the bioreactor has a tank containing a mixed solution of animal cells and the culture medium,
the external circulation renewal device of the culture medium comprising a ventilation component, wherein the ventilation component is arranged outside the tank and comprises a gas distributor, the gas distributor is configured to transfer a gas to the culture medium separated from the animal cells, and the gas distributor includes a shell and a diaphragm arranged in the shell, wherein the shell of the gas distributor includes a hollow inner cavity, the diaphragm separates the hollow inner cavity of the shell into a culture medium chamber through which the culture medium flows and a gas chamber through which the gas flows, and the diaphragm is configured to dissolve the gas in the gas chamber into the culture medium in the culture medium chamber without bubbles.

52. The external circulation renewal device of the culture medium according to claim 51, wherein the diaphragm is arranged to make the culture medium chamber and the gas chamber in a form of an inner cylinder and an outer cylinder nested with each other.

53. The external circulation renewal device of the culture medium according to claim 52, wherein the diaphragm is in a shape of a cylinder with both ends open and is serrated or wavy along a circumferential direction of the cylinder with both open ends.

54. The external circulation renewal device of the culture medium according to claim 52, wherein a top end of the diaphragm is fixedly connected to a top wall of the shell, and a bottom end is fixedly connected to a bottom wall of the shell.

55. The external circulation renewal device of the culture medium according to claim 52, wherein non-woven fabric is configured to adhere to the diaphragm to enhance strength of the diaphragm.

56. The external circulation renewal device of the culture medium according to claim 51, wherein the gas chamber comprises a ventilation slot arranged along an inner surface of a sidewall of the shell, and the ventilation slot is configured to guide the inlet gas to flow on the entire inner surface of the sidewall.

57. The external circulation renewal device of the culture medium according to claim 56, wherein the diaphragm is arranged on the inner surface of the sidewall and covers the ventilation slot.

58. The external circulation renewal device of the culture medium according to claim 56, wherein the sidewall comprises a plurality of convex parts, and the plurality of convex parts protrude radially inward from the inner surface of the sidewall.

59. The external circulation renewal device of the culture medium according to claim 58, wherein the plurality of convex parts form a support frame for supporting the diaphragm.

60. The external circulation renewal device of the culture medium according to claim 58, wherein the ventilation slot is formed in a space between the inner surface of the sidewall and side surfaces of adjacent convex parts.

61. The external circulation renewal device of the culture medium according to claim 58, wherein the diaphragm is fixed to a radial inner surface of the convex parts of the sidewall.

62. The external circulation renewal device of the culture medium according to claim 56, wherein the ventilation slot is in a serpentine shape winding mainly along a horizontal direction on the entire inner surface of the sidewall.

63. The external circulation renewal device of the culture medium according to claim 56, wherein the ventilation slot is in a serpentine shape winding mainly along a vertical direction on the entire inner surface of the sidewall.

64. The external circulation renewal device of the culture medium according to claim 56, wherein the ventilation slot is in a spiral shape extending from the bottom to the top around the entire inner surface of the sidewall.

65. The external circulation renewal device of the culture medium according to claim 51, wherein the culture medium chamber of the gas distributor is in fluid communication with the tank through a liquid outlet pipe.

66. The external circulation renewal device of the culture medium according to any one of claims 51-65, wherein the diaphragm uses a dense membrane or a microporous membrane.

67. The external circulation renewal device of the culture medium according to claim 66, wherein a pore size of the microporous membrane is configured to make the diaphragm ventilated without bubbles under a certain pressure.

68. The external circulation renewal device of the culture medium according to claim 67, wherein the pore size of the microporous membrane is less than 0.05 µm.

69. The external circulation renewal device of the culture medium according to claim 68, wherein the pore size of the microporous membrane is one or more of 0.01 µm, 0.05 um, 0.10 µm, 0.20 µm, and 0.04 µm.

70. The external circulation renewal device of the culture medium according to claim 66, wherein a thickness of the dense membrane is between 50 µm to 500 µm.

71. The external circulation renewal device of the culture medium according to any one of claims 51-65, wherein the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like.

72. The external circulation renewal device of the culture medium according to any one of claims 51-65, wherein the diaphragm is processed with hydrophilic and positive charge or negative charge treatment so that it is not easy to be blocked due to being adsorbed by animal cells.

73. The external circulation renewal device of the culture medium according to any one of claims 51-65, wherein the ventilation component further comprises a gas inlet unit and a gas outlet unit, wherein the gas inlet unit is in fluid communication with the gas chamber and transmits the inlet gas to the gas chamber, and the outlet unit is in fluid communication with the gas chamber and discharges an undissolved gas in the gas chamber from the gas distributor.

74. The external circulation renewal device of the culture medium according to claim 73, wherein the gas inlet unit is in fluid communication with the gas chamber through a gas inlet pipe and comprises a gas flow component regulator arranged on the gas inlet pipe, the gas flow component regulator being configured to adjust a component proportion of the inlet gas.

75. The external circulation renewal device of the culture medium according to claim 74, wherein the gas flow component regulator is one of a mass flowmeter or a gas-proportion regulating valve.

76. The external circulation renewal device of the culture medium according to claim 73, wherein the inlet gas includes air, oxygen, and carbon dioxide, or includes nitrogen, oxygen, and carbon dioxide.

77. The external circulation renewal device of the culture medium according to claim 73, wherein the gas outlet unit is in fluid communication with the gas chamber through a gas outlet pipe and comprises a pressure control valve and a sensor arranged in the gas outlet pipe to control gas pressure in the gas chamber.

78. The external circulation renewal device of the culture medium according to claim 77, wherein the gas pressure in the gas chamber is maintained between 0.01Mpa-0. 1Mpa.

79. The external circulation renewal device of the culture medium according to claim 74, wherein the ventilation component further comprises a dissolved oxygen electrode arranged in the mixed solution, and the dissolved oxygen electrode is configured to detect a dissolved oxygen concentration value in the mixed solution.

80. The external circulation renewal device of the culture medium according to claim 79, wherein a controller is configured to adjust a proportion of oxygen in the inlet gas of the gas flow component regulator through the dissolved oxygen concentration value detected by the dissolved oxygen electrode, so as to adjust the dissolved oxygen concentration value of the mixed solution in the tank.

81. The external circulation renewal device of the culture medium according to claim 74, wherein the ventilation component further comprises a pH electrode arranged in the mixed solution, and the pH electrode is configured to detect a pH value in the mixed solution.

82. The external circulation renewal device of the culture medium according to claim 81, wherein the controller is configured to adjust a proportion of carbon dioxide in the inlet gas of the gas flow component regulator through the pH value detected by the pH electrode, so as to adjust the pH value of the mixed solution.

83. The external circulation renewal device of the culture medium according to any one of claims 51-65, wherein the external circulation renewal device of the culture medium further comprises a cell separation device configured to separate the culture medium from animal cells.

84. The external circulation renewal device of the culture medium according to claim 83, wherein the cell separation device is arranged in the tank and is in fluid communication with the gas distributor.
